# EUROPÄISCHE PATENTSCHRIFT

(11) **EP 3 750 508 B1**
(45) Veröffentlichungstag und Bekanntmachung des Hinweises auf die Patenterteilung: **27.09.2023**
(21) Anmeldenummer: 20179189.4
(22) Anmeldetag: 10.06.2020
(51) Int. Cl.: A61F 2/00

(54) **VERFAHREN UND VORRICHTUNG ZUR IN-VITRO UNTERSUCHUNG DER WECHSELWIRKUNG VON BLUT MIT EINEM TESTOBJEKT**
METHOD AND DEVICE FOR THE IN-VITRO INVESTIGATION OF THE INTERACTION BETWEEN BLOOD AND A TEST OBJECT
PROCÉDÉ ET DISPOSITIF D'EXAMEN IN VITRO DE L'INTERACTION DU SANG AVEC UN OBJET D'ESSAI

(30) Priorität: 12.06.2019 DE 102019115940
(43) Veröffentlichungstag der Anmeldung: 16.12.2020
(73) Patentinhaber: Fraunhofer-Gesellschaft zur Förderung der angewandten Forschung e.V., 80686 München (DE)
(72) Erfinder: Kirschbaum, Michael, 80686 München (DE)
(74) Vertreter: v. Bezold & Partner Patentanwälte - PartG mbB

(56) Entgegenhaltungen:
- WO-A1-2005/033671
- WO-A1-2005/033671
- KILANI MOHAMMAD ET AL: "Further development on a gentle electromagnetic pump for fluids with stress-sensitive microparticles", SENSORS AND ACTUATORS A: PHYSICAL, ELSEVIER BV, NL, Bd. 247, 23. Juni 2016 (2016-06-23), Seiten 440-447, XP029713786, ISSN: 0924-4247, DOI: 10.1016/J.SNA.2016.06.031
- STEFAN SINN ET AL: "A novel in vitro model for preclinical testing of the hemocompatibility of intravascular stents according to ISO 10993-4", JOURNAL OF MATERIALS SCIENCE: MATERIALS IN MEDICINE, KLUWER ACADEMIC PUBLISHERS, BO, Bd. 22, Nr. 6, 21. Mai 2011 (2011-05-21), Seiten 1521-1528, XP019916464, ISSN: 1573-4838, DOI: 10.1007/S10856-011-4335-2
- C. Sperling ET AL: "Test methods for hemocompatibility of biomaterials" In: "Hemocompatibility of biomaterials for clinical applications : blood-biomaterials interactions", 3. November 2017 (2017-11-03), Elsevier, UK, XP055611656, ISBN: 978-0-08-100497-5 Seiten 77-104, DOI: 10.1016/B978-0-08-100497-5.00005-7, * Absätze [4.3.6.1], [4.3.6.2]; Abbildungen 4.7, 4.8 *

## Beschreibung

Die Erfindung betrifft ein Verfahren zur in-vitro Untersuchung der Wechselwirkung von Blut mit einem Testobjekt, insbesondere einem kardiovaskulären Implantat oder einem Medizinprodukt. Außerdem wird eine Vorrichtung zur in-vitro Untersuchung der Wechselwirkung von Blut mit einem Testobjekt bereitgestellt.

In der vorliegenden Beschreibung der Erfindung werden folgende Dokumente des Standes der Technik zitiert:
[1] W. van Oeveren, I.F.J. Tielliu, J. de Hart in "International Journal of Biomaterials", Bd. 2012, Article ID 673163 (Mai 2012);
[2] W. van Oeveren in "Scientifica", Bd. 2013, Article ID 392584, S. 1-14 (2013);
[3] U. Streller, C. Sperling, H. Hübner, R. Hanke, C. Werner in "Journal of Biomedical Materials Research Part B Applied Biomaterials", Bd. 66B, S. 379-390 (2003);
[4] J. W. Mulholland, J. C. Shelton, X. Y. Luo in "Journal of Fluids and Structures", Bd. 20, Nr. 1, S. 129-140 (January 2005);
[5] G. E. Engels, S. L. J. Blok, W. van Oeveren in "Biointerphases", Bd. 11, Nr. 3, 031004 (September 2016);
[6] P. Borgdorff, R. H. van den Berg, M. A. Vis, G. C. van den Bos, G. J. Tangelder in "The Journal of Thorac Cardiovascular Surgery", Bd. 118, Nr. 5, S. 946-52 (1999);
[7] A. B. Chandler in "Laboratory Investigation J Tech Methods Pathol." Bd. 7, Nr. 2, S. 110-114 (1958);
[8] S. Braune, M. Grunze, A. Straub, F. Jung in "Biointerphases", 8:33, (Dezember 2013);
[9] S. Braune, A. Lendlein, F. Jung in "Hemocompatibility of Biomaterials for Clinical Applications", 1. Auflage, Kapitel: Teile 1-3, S. 51-76 (November 2017);
[10] S. Sinn, T. Scheuermann, S. Deichelbohrer, G. Ziemer, H. P. Wendel in "Journal of Materials Science: Materials in Medicine", Bd. 22, Nr. 6, S. 1521-1528 (Juni 2011);
[11] WO 2005/033671 A1;
[12] JP 2007/089974 A;
[13] US 4,627,419 A;
[14] US 6,066,085 A;
[15] US 2010/0204539 A1;
[16] US 6,579,223 B2; und
[17] M. Kilani et al. in "Sensors and Actuators" A 247 ( 2016 ) 440-447.

Die in-vitro Untersuchung der Blutverträglichkeit (BV) kardiovaskulärer Implantate und Medizinprodukte ist wesentlicher Bestandteil ihrer Entwicklung und Optimierung. Von besonderem Interesse sind strömungsabhängige Prozesse oder strömungsabhängige Parameter, wie die Parameter Hämolyse (Auflösung roter Blutkörperchen bzw. Erythrozyten), Aktivierung von Thrombozyten (Blutplättchen- oder Platelet-Aktivierung) oder der Degradierung des von-Willebrand-Faktors (Trägerprotein des Blutgerinnungsfaktors VIII), wie z. B. in [1-3] beschrieben. Diese Prozesse werden vorrangig durch die geometrischen Eigenschaften eines Testobjekts, insbesondere eines kardiovaskulären Implantats, und die Strömungsbedingungen im Blutgefäß bestimmt. Um den Effekt des Testobjekts auf Blut qualitativ und/oder quantitativ zu erfassen, sollten scherratenabhängige Einflüsse durch den Untersuchungsapparat möglichst unterdrückt werden.

Für sehr kleine Testobjekte, z.B. kardiovaskuläre Implantate wie die millionenfach implantierten und sich in ständiger Weiterentwicklung befindlichen Stents (Gefäßstützen), besteht ein Interesse an miniaturisierten Testumgebungen mit kleinen Blutvolumina. In kleinen Volumina sind messbare Marker, welche durch strömungsabhängige Prozesse im Blut induziert werden, in einer Konzentration vorhanden, die ihre Bestimmung erleichtert. Bekannte Systeme bzw. Pumpmechanismen sind zur dynamischen in-vitro Untersuchung der Wechselwirkung von Blut mit dem Testobjekt, z.B. dem Stent, nur beschränkt geeignet, da sie keine dynamische Untersuchung unter kontrollierten und physiologischen Strömungsbedingungen und/oder mit ausreichender Reproduzierbarkeit erlauben. Die strömungstechnische Optimierung der Geometrie eines kardiovaskulären Implantats erfolgt daher in der Regel nur auf Grundlage von numerischen Simulationen. Beispielsweise wird in [4] und [5] jeweils die Strömungsdynamik zweier unten näher beschriebener herkömmlich verwendeter Testvorrichtungen, der Rollerpumpe (Quetschpumpe) und des so genannten "Hemobiles", zur dynamischen Druckveränderung numerisch untersucht.

Herkömmliche Vorrichtungen zur dynamischen in-vitro Untersuchung der Blutverträglichkeit oder Hämokompatibilität von Testobjekten, beispielsweise kardiovaskulären Implantaten, sind typischerweise mit einem Schlauch ausgestattet, in dem das Testobjekt fest angeordnet ist und durch den Blut strömt. Dabei kann entweder der Schlauch stationär fixiert und eine Blutströmung im Schlauch erzeugt werden, oder die Blutströmung kann durch eine Bewegung des Schlauchs erzeugt werden.

Bei der in [4] beschriebenen Technik wird als Pumpe eine herkömmliche Rollerpumpe (Quetschpumpe) verwendet. Von Nachteil ist, dass auf einen stationären Schlauch wirkende Rollen Scherkräfte induzieren, welche das durch den Schlauch getriebene Blut schädigen und somit die Testergebnisse zur Blutverträglichkeit des Testobjekts verfälschen.

Der in [7, 10] beschriebene sogenannte "Chandler-Loop" ist ein etwa zur Hälfte mit Blut befüllter Kunststoffschlauch, in dem das Testobjekt fixiert ist und der zu einem Ring geschlossen und in Rotation versetzt wird, wodurch das Testobjekt periodisch durch das Blut gezogen wird. Diese Technik hat jedoch den Nachteil, dass das Testobjekt bei jeder Umdrehung im Gegensatz zu physiologischen Bedingungen im Körper mit Luft in Kontakt kommt. Durch den Kontakt mit Luft können Reaktionen ausgelöst werden, die das Testergebnis bezüglich einer Blutschädigung durch Scherkräfte verfälschen. Insbesondere kommt es durch den im System notwendigen Luftraum während der Rotation zu Schaumbildung und Verwirbelungen, wodurch unkontrollierte Strömungsbedingungen herrschen und nur bedingt reproduzierbare und/oder nur mit hohen Streubreiten belegte Daten erzeugt werden können. Zudem wird die maximal erreichbare Strömungsgeschwindigkeit insbesondere bei kleinen Testobjekten und/oder bei geringen Schlauchdurchmessern stark durch Kapillarkräfte limitiert, weshalb beispielsweise für Stents in der Regel keine physiologischen Strömungsraten appliziert werden können. Die Kapillarkräfte können bewirken, dass sich das Blut bei zu hohen Umdrehungsgeschwindigkeiten mit dem Schlauch bewegt, so dass keine Relativbewegung zwischen Testobjekt und Blut mehr stattfindet.

Das in [1,5] beschriebene "Hemobile" ist wie der "Chandler-Loop" mit einem geschlossenen Schlauch ausgestattet, der jedoch mittels einer in [11] beschriebenen Vorrichtung in Rotation mit wechselnder Drehrichtung versetzt wird. Beim "Hemobile" wird der Schlauch vollständig, d.h. ohne Luftraum, mit Blut gefüllt und ein Kugelventil eingebaut, das einen Rückfluss des Blutes bei Umkehr der Drehrichtung des Schlauchs verhindert. Von Nachteil ist, dass das Kugelventil das durchströmende Blut durch Scherkräfte schädigt und die Testergebnisse zur Untersuchung der Wechselwirkung von Blut und Testobjekt verfälscht. Ferner ist wie beim "Chandler-Loop" die maximal erreichbare Strömungsgeschwindigkeit aufgrund von Kapillarkräften bei dünnen Schläuchen begrenzt.

In [6] wird die Erzeugung einer Strömung aufgrund hydrostatischer Kräfte oder der Gravitationskraft beschrieben, indem die Enden eines Schlauchs wechselweise angehoben und abgesenkt werden. Die durch die Schwerkraft erzeugte Strömungsgeschwindigkeit ist jedoch bei einer im Labor üblichen Höhendifferenz beim Anheben und Absenken der Schlauchenden von beispielsweise 30 cm viel zu niedrig, um physiologische Strömungen zu simulieren. Bedeutend größere Höhendifferenzen würden zu hohe Blutvolumina zur Untersuchung erfordern.

Es sind auch in-vivo Blutpumpen bekannt. Diese gliedern sich auf in zwei Klassen zum extrakorporalen Einsatz in Herz-Lungen-Maschinen, beispielsweise während einer Herzoperation, und zur Implantation. In [12] wird das externe Pumpen von Blut mittels eines Laufrads beschrieben, welches jedoch das Blut beeinträchtigende Scherkräfte erzeugt. In [13] wird ein extrakorporales Pumpen mit einer Kombination aus Schwerkraft, die durch Rotation einer teils mit Blut und teils mit einer inaktiven Flüssigkeit gefüllten Fluidkammer entsteht, und dem Pumpen der inaktiven Flüssigkeit beschrieben. Implantierte in-vivo Blutpumpen in [14-16] erzeugen jeweils einen relativ zur Strömungsrichtung des Blutes seitlichen Druck auf eine Membran, was in [14] mittels einer Platte und in [15] und [16] fluidgetrieben erfolgt. Zum Gebrauch als Testvorrichtungen zur in-vitro Untersuchung der Wechselwirkung von Blut mit einem Testobjekt sind die oben beschriebenen in-vivo Blutpumpen ungeeignet.

Die Robustheit und Präzision der herkömmlichen Vorrichtungen zur in-vitro Untersuchung von Wechselwirkungen zwischen Blut und einem Testobjekt sind nicht ausreichend, um feine Unterschiede in der Geometrie oder der Oberflächenbeschaffenheit von Testobjekten, insbesondere Stents, unter physiologischen Strömungsbedingungen auflösen und auf dieser Grundlage eine sorgfältige Optimierung der Implantat-Geometrie durchführen zu können. Ferner fehlen - wie in [8,9] zusammengefasst - Standards für in-vitro Untersuchungen der Wechselwirkung von Blut mit Testobjekten, was die Vergleichbarkeit von Testergebnissen und die Entwicklung optimierter blutverträglicher Produkte behindert.

Als Testobjekte für in-vitro Untersuchungen sind nicht nur kardiovaskuläre Implantate, wie beispielsweise Stents, sondern auch Medizinprodukte, beispielsweise Katheter, von Interesse.

Aus [17] ist ein Verfahren zum schonenden Pumpen von scherstressempfindlichen Flüssigkeiten bekannt, bei dem in einer Flüssigkeitsleitung ein Kolben angeordnet und mit einem äußeren Magnetfeld bewegt wird. Der Kolben schiebt die Flüssigkeit in der Flüssigkeitsleitung vor sich her.

Die Aufgabe der Erfindung ist es, ein verbessertes Verfahren und/oder eine verbesserte Vorrichtung zur in-vitro Untersuchung der Wechselwirkung von Blut mit einem Testobjekt bereitzustellen, mit denen Nachteile herkömmlicher Techniken vermieden werden. Insbesondere soll die Veränderung von Blutkomponenten durch etwaige Scherkräfte in der Vorrichtung, die ohne das Testobjekt auftreten könnten, minimiert werden. Alternativ oder ergänzend besteht die Aufgabe, eine leicht implementierbare, parallelisierbare und/oder kostengünstige in-vitro Untersuchung der Wechselwirkung von Blut mit einem Testobjekt bereitzustellen, bei deren Anwendung nur geringe Mengen an Blut zur Untersuchung benötigt werden. Eine weitere Aufgabe besteht darin, die in-vitro Untersuchung der Wechselwirkung von Blut mit einem Testobjekt so zu verbessern, dass präzise Messdaten mit geringen Streubreiten erhalten werden.

Diese Aufgabe wird durch ein Verfahren und/oder eine Vorrichtung zur in-vitro Untersuchung der Wechselwirkung von Blut mit einem Testobjekt mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Aspekt der Erfindung wird die obige Aufgabe durch ein Verfahren zur in-vitro Untersuchung der Wechselwirkung von Blut mit einem Testobjekt gelöst. Das Verfahren umfasst den Schritt der Bereitstellung des Testobjekts und des Blutes in einer Behältereinrichtung, wobei das Testobjekt in der Behältereinrichtung fixiert wird und das Blut als eine Blutsäule entlang einer Längsrichtung (Längsausdehnung) der Behältereinrichtung angeordnet wird. Ferner umfasst das Verfahren die Erzeugung einer Blutströmung in der Behältereinrichtung für eine vorbestimmte Expositionszeit mittels einer Druckerzeugungseinrichtung, wobei die Blutströmung am Testobjekt vorbeitritt und/oder durch eine Öffnung des Testobjekts durchtritt. Das Verfahren umfasst des Weiteren die Untersuchung des Blutes zur Ermittlung mindestens eines Blutparameters, der von einer Wechselwirkung des Blutes mit dem Testobjekt abhängig ist.

Die Druckerzeugungseinrichtung umfasst mindestens einen Druckerzeuger, mit dem in der Behältereinrichtung ein Überdruck oder ein Unterdruck relativ zum Atmosphärendruck oder relativ zu einem weiteren, mit der Behältereinrichtung verbundenen Druckerzeuger erzeugt werden kann. Erfindungsgemäß ist vorgesehen, dass mit der Druckerzeugungseinrichtung an mindestens einem Ende der Behältereinrichtung von außen ein gezielt einstellbarer, veränderlicher Druck auf die Blutsäule ausgeübt wird, so dass die Blutströmung mit einer alternierenden Strömungsrichtung entlang der Längsrichtung der Behältereinrichtung erzeugt wird. Die Ausübung eines äußeren Drucks (Über- oder Unterdruck relativ zum Atmosphärendruck) auf die Blutsäule bedeutet, dass der von der Druckerzeugungseinrichtung angewendete Druck auf mindestens ein Ende der Blutsäule in der Behältereinrichtung wirkt.

Gemäß einem weiteren allgemeinen Aspekt wird die oben genannte Aufgabe durch eine Vorrichtung, die zur in-vitro Untersuchung der Wechselwirkung von Blut mit einem Testobjekt eingerichtet ist, gelöst. Die Vorrichtung umfasst eine Behältereinrichtung, die dazu ausgebildet ist, das zu untersuchende Testobjekt in einer fixierten Anordnung und ein Testvolumen von Blut in Form eine Blutsäule entlang einer Längsrichtung der Behältereinrichtung aufzunehmen. Die Vorrichtung umfasst ferner eine Druckerzeugungseinrichtung, die dazu ausgebildet ist, in der Behältereinrichtung eine Blutströmung zu erzeugen, die am Testobjekt vorbeitritt und/oder durch eine Öffnung des Testobjekts durchtritt.

Erfindungsgemäß weist die Behältereinrichtung zwei Enden (im Folgenden auch als erstes Ende bzw. erste Enden und zweites Ende bzw. zweite Enden bezeichnet) auf, wobei die Druckerzeugungseinrichtung mit mindestens einem der Enden druckdicht gekoppelt ist. Ferner ist die Druckerzeugungseinrichtung erfindungsgemäß dazu ausgebildet, an mindestens einem der Enden der Blutsäule einen äußeren, veränderlichen Druck auf die Blutsäule auszuüben, so dass die Blutströmung mit einer alternierenden Strömungsrichtung entlang der Längsrichtung der Behältereinrichtung erzeugt werden kann.

Mit der erfindungsgemäß vorgesehenen Druckerzeugung und Druckapplikation von außen ist vorteilhafterweise der Druck an mindestens einem Ende der Blutsäule und mit diesem die Strömungsgeschwindigkeit des Blutes gezielt einstellbar. Abweichend von der herkömmlichen Technik z. B. gemäß [6], bei der die Strömungsgeschwindigkeit des Blutes durch die Länge der Blutsäule bestimmt wird und nur in einem begrenzten Bereich variierbar ist, wird erfindungsgemäß ein frei wählbarer äußerer Druck ausgeübt, d. h. es steht ein zusätzlicher Freiheitsgrad bei der Einstellung der Strömungsgeschwindigkeit des Blutes unabhängig von der Länge der Blutsäule und selbst bei einem geringen Innendurchmesser der Behältereinrichtung zur Verfügung. Die Strömungsgeschwindigkeit ist in einem erweiterten Bereich und mit erhöhter Genauigkeit reproduzierbar einstellbar. Die Vergleichbarkeit von gleichzeitigen oder aufeinander folgenden Mehrfach-Messungen steigt, und es sind Standards zur Charakterisierung der Blutverträglichkeit, insbesondere anhand der eingestellten Strömungsgeschwindigkeit und des Innendurchmessers der Behältereinrichtung, definierbar. Vorteilhafterweise hat die erfindungsgemäße Vorrichtung einen Aufbau, der mit kostengünstigen Komponenten realisierbar ist.

Blutverträglichkeits-Tests sind vor dem Hintergrund immer höher werdender Kosten und Fallzahlen von Blutkreislauferkrankungen auch in Zukunft für die Weiterentwicklung kardiovaskulärer Implantate und Medizinprodukte von essentieller Bedeutung. Auf Grundlage des erfindungsgemäßen Verfahrens und der zugehörigen Vorrichtung zur in-vitro Untersuchung der Wechselwirkung von Blut mit einem Testobjekt können beispielsweise für die weltweit ca. 100 Stent-herstellenden Unternehmen robuste und leicht parallelisierbare Tests für ihre Produkte bereitgestellt werden. Der Vorteil für die Anwender ergibt sich aus der Möglichkeit zur kostengünstigen Optimierung ihrer Produkte hinsichtlich blutverträglicher Strömungseigenschaften.

Die Behältereinrichtung umfasst mindestens einen Objektaufnahmebehälter, beispielsweise einen Kunststoffschlauch. Der mindestens eine Objektaufnahmebehälter hat eine längliche Form mit jeweils einer Öffnung an jedem Ende zur Druckausübung und/oder zum Druckausgleich. Eine Blutsäule kann beispielsweise in einer Behältereinrichtung in Gestalt eines im Wesentlichen U-förmigen Schlauchs erzeugt werden. Die Enden der Blutsäule können sich in den beiden nach oben (entgegengesetzt zur Gravitationsrichtung) weisenden Schenkeln des U-förmigen Schlauchs befinden. Bei geeigneter Abdichtung der Enden des mindestens einen Objektaufnahmebehälters ist alternativ oder zusätzlich mindestens ein Objektaufnahmebehälter mit einer geraden Form oder mit einer gekrümmten Form mit nach unten (in Gravitationsrichtung) weisenden Enden verwendbar.

Die Behältereinrichtung bzw. der Objektaufnahmebehälter umfasst vorzugsweise ein nicht dehnbares Material, das durch den äußeren, veränderlichen Druck nicht oder nur vernachlässigbar gering deformiert wird. Alternativ kann das Material als nicht oder nur mäßig elastisch bezeichnet werden.

Das Blut kann zum Beispiel durch vorhandenes und/oder für andere Zwecke einem Probanden gesondert entnommenes Frischblut oder optional aus einer Blutkonserve bereitgestellt werden. Für den Test wird ein geringes Blutprobevolumen verwendet, beispielsweise im Bereich von 0,5 ml bis 10 ml. Die Entnahme des Blutes von dem Probanden ist nicht Teil des erfindungsgemäßen Verfahrens.

Die Behältereinrichtung weist vorteilhafterweise entlang der Längsausdehnung eine Länge auf, die mindestens so lang ist, dass das Testobjekt zu jedem Zeitpunkt der Blutströmung mit alternierender Strömungsrichtung von Blut vollständig benetzt ist. Die Länge der Behältereinrichtung beträgt bevorzugt zwischen 20 cm und 100 cm. Der Innendurchmesser der Behältereinrichtung beträgt vorzugsweise zwischen 2 mm und 10 mm. Das Fassungsvolumen der Behältereinrichtung beträgt vorzugsweise zwischen 0,5 ml und 10 ml.

Die wechselweise äußere Druckerzeugung an einem oder beiden Enden der Blutsäule bewirkt, dass das Blut im Inneren der Blutsäule das Testobjekt scherratenarm umströmt und/oder durchströmt. Das Testobjekt ist vorteilhafterweise zu jedem Zeitpunkt der Blutströmung mit alternierender Strömungsrichtung von Blut benetzt. Vorzugsweise ist vorgesehen, dass das Testobjekt während der Dauer der Erzeugung der Blutströmung nicht in Kontakt mit Luft oder einem Druckfluid gebracht wird. Durch das Anlegen eines äußeren, veränderlichen Drucks können ferner physiologische Strömungsgeschwindigkeiten, beispielsweise zwischen 100 mm/s und 400 mm/s oder darüber hinaus, typischerweise jedoch nicht größer als 2000 mm/s, erreicht werden. Die Druckveränderung an einem oder beiden Enden des Objektaufnahmebehälters oder der Behältereinrichtung kann mit einer festen Frequenz, beispielsweise zwischen 1 Hz und 2 Hz, wiederholt werden.

Das Verfahren wird vorzugsweise bei konstanter Temperatur durchgeführt. Beispielsweise kann die Behältereinrichtung in einem Thermo-Schrank temperiert sein.

Der Druck wird gemäß einem bevorzugten Ausführungsbeispiel wechselweise an beiden Enden als Überdruck ausgeübt. Durch das wechselweise Anlegen eines Überdrucks an beiden Enden der Blutsäule können vorteilhafterweise physiologische Strömungsgeschwindigkeiten in beiden Strömungsrichtungen relativ zu dem Testobjekt erreicht werden. Ferner kann durch das wechselweise Anlegen von Überdruck an beiden Enden vorteilhafterweise die Periodendauer der Druckänderung gegenüber einem nur einseitig anlegbaren Überdruck verkürzt werden. Vorteilhaft ist auch, dass durch das wechselweise Anlegen von Überdruck ein Unterdruck, insbesondere an den Enden der Blutsäule, vermieden und so Ausgasungen und Blasenbildungen minimiert werden.

Vorteilhafterweise sind verschiedene Typen von Druckerzeugern verfügbar, welche die Druckerzeugungseinrichtung bilden. Druckerzeuger umfassen z. B. Druckfluidquellen und/oder Druckkolben. Der Druck wird gemäß der Erfindung durch die Druckerzeugungseinrichtung an mindestens einem der Enden der Behältereinrichtung durch die Applikation eines auf das Blut wirkenden Druckgases, insbesondere Luft oder ein Edelgas, und/oder flüssigen Fluids ausgeübt. Bei dem Ausführungsbeispiel mit dem wechselweisen Anlegen eines Überdrucks an beiden Enden der Blutsäule sind zwei Druckkolben vorgesehen. Die Druckkolben und die Blutsäule sind durch einen Puffer getrennt, beispielsweise durch das nicht mit dem Blut wechselwirkende, flüssige oder gasförmige Fluid (inertes Fluid). Der Puffer ist das oben genannte Druckgas und/oder das inertes Fluid, dessen Dichte geringer ist als die Dichte von Blut. Aufgrund der geringeren Dichte befindet sich das inerte Fluid direkt oberhalb der Enden der Blutsäule. Gemäß einer weiteren Variante des Verfahrens und der zugehörigen Vorrichtung kann eine Druckfluidquelle, wie z. B. ein Druckgasreservoir, mit einer Ventilsteuerung zur Erzeugung eines wechselweisen Überdrucks an den beiden Enden der Behältereinrichtung vorgesehen sein.

Die Behältereinrichtung kann einen einzigen Objektaufnahmebehälter mit einem ersten und einem entgegengesetzten zweiten Ende umfassen. Alternativ kann die Behältereinrichtung mehrere Objektaufnahmebehälter umfassen, die jeweils ein erstes und ein entgegengesetztes zweites Ende aufweisen. In einem ersten Ausführungsbeispiel mit mehreren, parallel geschalteten Objektaufnahmebehältern wirkt die Druckerzeugungseinrichtung auf alle Objektaufnahmebehälter gemeinsam, so dass an den ersten oder den zweiten Enden aller Objektaufnahmebehälter jeweils der gleiche Druck ausgeübt wird. In diesem Fall sind die ersten Enden und die zweiten Enden jeweils miteinander, z. B. über eine Verbindungsleitung, verbunden. In einem alternativen zweiten Ausführungsbeispiel mit mehreren, nebeneinander angeordneten Objektaufnahmebehältern wirkt die Druckerzeugungseinrichtung auf jeden Objektaufnahmebehälter einzeln, so dass an jedem Objektaufnahmebehälter ein individueller Druck ausgeübt wird. Die Länge, der Durchmesser und das Volumen jedes Objektaufnahmebehälters können den oben genannten Größen der Behältereinrichtung entsprechen.

Der Druckerzeuger der Druckerzeugungseinrichtung in Gestalt des Druckkolbens kann mindestens eine Kolbenspritze umfassen. Bei parallel geschalteten Objektaufnahmebehältern wirkt die Kolbenspritze gemäß einem ersten Ausführungsbeispiel gleichzeitig auf die ersten Enden aller Objektaufnahmebehälter. Bei dem Ausführungsbeispiel mit dem wechselweisen Anlegen eines Überdrucks an beiden Enden der Behältereinrichtung sind eine erste Kolbenspritze und eine zweite Kolbenspritze vorgesehen, die jeweils entsprechend auf die ersten und die zweiten Enden der Behältereinrichtung wirken. Gemäß einem weiteren Ausführungsbeispiel kann die Druckerzeugungseinrichtung mehrere erste und/oder zweite Kolbenspritzen umfassen, die jeweils entsprechend einzeln auf die ersten und/oder die zweiten Enden der Objektaufnahmebehälter wirken.

Das Testobjekt kann beispielsweise ein kardiovaskuläres Implantat oder ein Medizinprodukt sein. Das Implantat kann insbesondere ein Stent sein. Das Medizinprodukt kann insbesondere ein Katheter sein.

Die Expositionszeit (Dauer der Erzeugung einer Blutströmung) beträgt vorteilhafterweise mindestens eine Stunde und/oder höchstens sechs Stunden. Besonders bevorzugt wird die Expositionszeit im Bereich von zwei bis drei Stunden gewählt. Durch eine Expositionszeit von mindestens einer Stunde werden kurzzeitige Effekte, insbesondere eine reduzierte Funktion von Thrombozyten, eliminiert, welche durch den Umfüllvorgang des Blutes in die Behältereinrichtung - insbesondere auch eine Behältereinrichtung ohne Testobjekt - hervorgerufen werden. Solche kurzzeitigen Effekte würden die Testergebnisse hinsichtlich der zu bestimmenden Wechselwirkung von Blut mit dem Testobjekt verfälschen. Ferner sollte die Expositionszeit sechs Stunden nicht überschreiten, da nach spätestens diesem Zeitraum üblicherweise Veränderungen von Blut außerhalb eines lebenden Körpers auftreten und daraus eine Verfälschung der Testergebnisse resultiert. Die maximale Expositionszeit der erfindungsgemäßen Vorrichtung übersteigt die maximale Expositionszeit von vier Stunden herkömmlicher Vorrichtungen, z.B. des "Chandler-Loops" oder der Rollerpumpe (Quetschpumpe), deren Luftkontakt und/oder mechanische Einwirkung durch einen seitlichen Druck auf den Schlauch die Blutbestandteile frühzeitig schädigt.

Der mindestens eine Blutparameter, der vorzugsweise nach der Expositionszeit erfasst wird, ist bevorzugt mindestens einer der Parameter Hämolyse, Degradierung des von-Willebrand-Faktors und Aktivierung von Thrombozyten. Alternativ oder ergänzend kann ein, beispielsweise kleines oder großes, Blutbild (Hämogramm) erstellt werden. Das Blutbild kann eine Anzahl von Leukozyten, Erythrozyten, Hämoglobin, Thrombozyten und unspezifischen Entzündungsmarkern enthalten. Alternativ kann der Anteil an Hämatokrit und/oder das mittlere Thrombozyten-Volumen bestimmt werden.

Gemäß einer weiteren Variante der Erfindung kann das Verfahren den Schritt der Untersuchung des Testobjekts zur Ermittlung einer Belegungsdichte des Testobjekts mit Blutbestandteilen nach der Expositionszeit umfassen. Die Belegungsdichte mit Blutbestandteilen kann z. B. durch eine Anlagerung von Thrombozyten und/oder Proteinen gebildet werden. Das Testobjekt kann nach Abschluss des Verfahrens außerhalb der Behältereinrichtung mittels Färbemethoden und/oder Spektroskopie untersucht werden. Alternativ oder ergänzend kann das Testobjekt und/oder das Blut während der Expositionszeit in situ mittels einer Kamera und/oder einem Mikroskop und/ oder mit immunbiochemischen Verfahren untersucht werden.

Gemäß einem weiteren bevorzugten Ausführungsbeispiel weist die Behältereinrichtung, insbesondere der mindestens eine Objektaufnahmebehälter, quer zu der Längsausdehnung eine Innenquerschnittsdimension auf, die an eine Außenquerschnittsdimension des Testobjekts angepasst ist. Ein kardiovaskuläres Implantat, wie z. B. ein Stent, kann in einem zusammengefalteten Zustand in den Objektaufnahmebehälter eingebracht und in diesem mit einem Ballonkatheter entfaltet werden, so dass das Implantat an den Wänden des Objektaufnahmebehälters fixiert wird. Alternativ oder ergänzend kann das Testobjekt in der Behältereinrichtung oder dem mindestens einen Objektaufnahmebehälter mittels einer Festhaltevorrichtung, wie z. B. einer von außen durch die Wand des Objektaufnahmebehälters eingeführten Nadel, fixiert sein. Die Behältereinrichtung kann zur Aufnahme des Testobjekts in Gestalt eines kardiovaskulären Implantats oder eines Medizinprodukt eingerichtet sein.

Die Behältereinrichtung oder der mindestens eine Objektaufnahmebehälter umfasst vorzugsweise einen geraden oder U-förmig gebogenen Schlauch oder ein gerades oder U-förmig gebogenes Rohr, insbesondere aus einem Kunststoff. Der Querschnitt des mindestens einen Objektaufnahmebehälters kann rund, oval oder polygonal sein. Die U-Form des mindestens einen Objektaufnahmebehälters mit nach oben gerichteten Schenkeln des U's kann sicherstellen, dass die Blutsäule von der Schwerkraft innerhalb der Behältereinrichtung oder dem Objektaufnahmebehälter gehalten wird. Das Material des mindestens einen Objektaufnahmebehälters ist vorzugsweise blutverträglich. Alternativ oder ergänzend kann der mindestens eine Objektaufnahmebehälter mit einem blutverträglichen Material beschichtet sein. Blutverträgliches Material zeichnet sich dadurch aus, dass Blut bei Kontakt mit dem Material unverändert bleibt oder vernachlässigbar wenig verändert wird, und es umfasst z. B. einen Kunststoff, wie Polyvinylchlorid (PVC) oder Silikon, und/oder eine Oberflächenbeschichtung aus Heparin, Polyethylenglykol (PEG) oder zwitterionischen Molekülen (z.B. Sulfobetaine).

Der mindestens eine Objektaufnahmebehälter umfasst vorzugsweise ein mäßig elastisches oder ein unelastisches Material. Dieses Material ist bevorzugt so gewählt, dass ein Vortrieb der Blutsäule nicht oder nur geringfügig, beispielsweise um weniger als 10 %, durch eine Erweiterung der Objektaufnahmebehälterwand beeinflusst wird. Das unelastische oder mäßig elastische Material des mindestens einen Objektaufnahmebehälters kann ferner biegsam sein, so dass ein Verbiegen zum Einbringen des Testobjekts und zum Anschluss mindestens eines Endes an eine Druckerzeugungseinrichtung ermöglicht ist.

Weitere Einzelheiten und Vorteile der Erfindung werden im Folgenden unter Bezug auf die beigefügten Zeichnungen beschrieben. Es zeigen:
- Figur 1: eine schematische Teilseitenansicht eines Ausführungsbeispiels einer Vorrichtung zur invitro Untersuchung der Wechselwirkung von Blut mit einem Testobjekt;
- Figur 2: eine perspektivische Teilansicht eines alternativen Ausführungsbeispiels der erfindungsgemäßen Vorrichtung mit mehreren Objektaufnahmebehältern;
- Figur 3: eine perspektivische Teilansicht eines weiteren alternativen Ausführungsbeispiels der erfindungsgemäßen Vorrichtung mit mehreren Objektaufnahmebehältern;
- Figur 4: eine schematische Ansicht eines Objektaufnahmebehälters mit einem Testobjekt in einer ersten Betriebsphase;
- Figur 5: eine schematische Ansicht des Objektaufnahmebehälters mit einem Testobjekt gemäß Figur 4 in einer zweiten Betriebsphase; und
- Figur 6: eine schematische Darstellung einer Druckerzeugungseinrichtung.

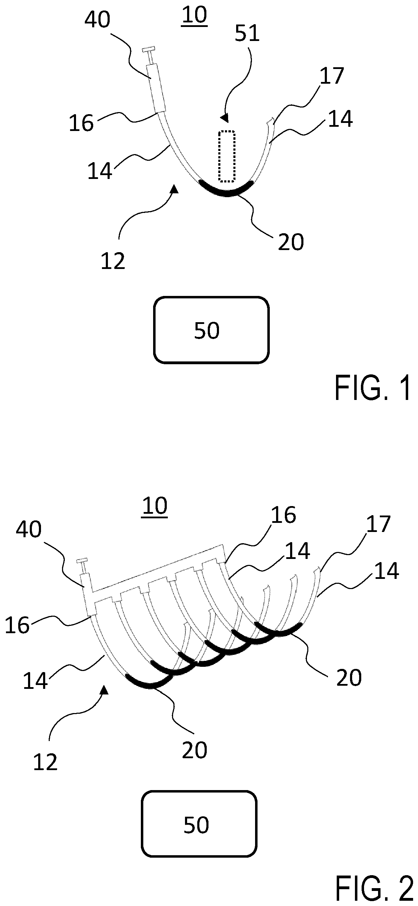

Die Figuren 1 bis 3 zeigen schematisch Teilansichten einer erfindungsmäßigen Vorrichtung 10 zur in-vitro Untersuchung der Wechselwirkung von Blut mit einem Testobjekt. Weitere Komponenten der Vorrichtung 10, wie z. B. eine Steuereinrichtung, insbesondere zur Steuerung der Druckerzeuger der Druckerzeugungseinrichtung, oder Einzelheiten von Untersuchungseinrichtungen 50, 51 sind nicht illustriert.

Die Vorrichtung 10 umfasst eine Behältereinrichtung 12 und eine Druckerzeugungseinrichtung 40 mit mindestens einem Druckerzeuger (z. B. die illustrierte Kolbenspritze) mindestens an einem ersten Ende 16 der Behältereinrichtung 12. Im Ausführungsbeispiel gemäß Figur 1 umfasst die Behältereinrichtung 12 einen einzigen Objektaufnahmebehälter 14, in dem sich eine Blutsäule 20 befindet. Bevorzugt ist der Objektaufnahmebehälter 14 U-förmig gebogen mit den nach oben weisenden, ersten und zweiten Enden 16 und 17. Die Blutsäule 20 wird durch die Schwerkraft in der Mitte des Objektaufnahmebehälters 14 gehalten. Der Objektaufnahmebehälter 14 umfasst vorzugsweise ein blutverträgliches und mäßig elastisches Material. Beispielsweise ist der Objektaufnahmebehälter aus Polyvinylchlorid (PVC) oder Silikon gebildet. Die Druckerzeugungseinrichtung 40, die druckdicht mit dem ersten Ende 16 des Objektaufnahmebehälters 14 verbunden ist, ist dazu ausgebildet, einen Überdruck zu erzeugen. An dem zweiten Ende 17 des Objektaufnahmebehälters 14 kann sich ein weiterer Druckerzeuger der Druckerzeugungseinrichtung (in Figur 1 nicht gezeigt) befinden, der dazu ausgebildet ist, an dem zweiten Ende 17 einen Überdruck zu erzeugen.

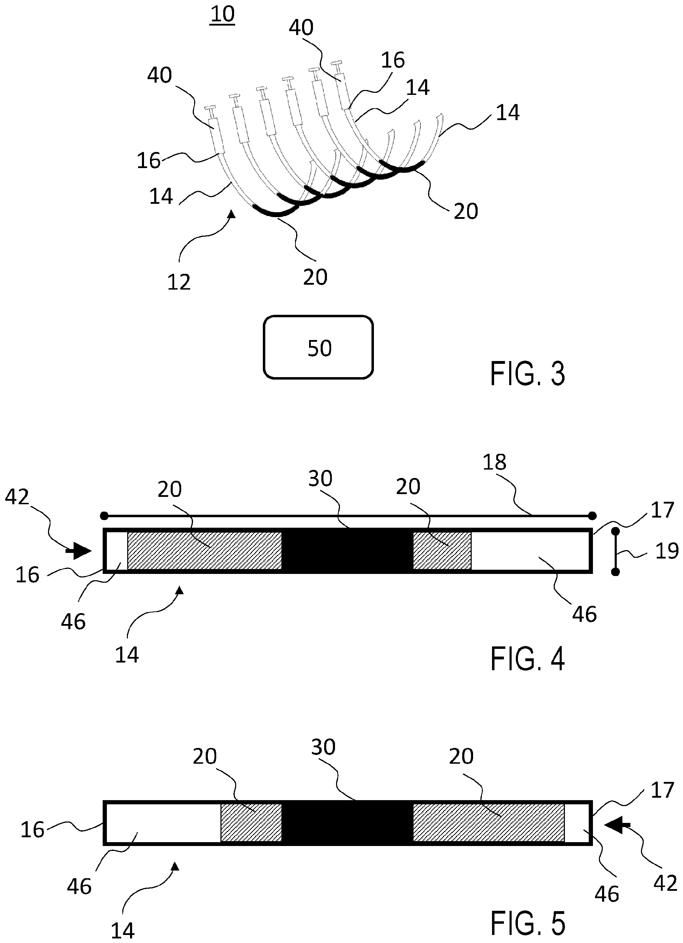

Gemäß einem Ausführungsbeispiel des Verfahrens zur in-vitro Untersuchung der Wechselwirkung von Blut mit einem Testobjekt wird ein Testobjekt 30, beispielsweise ein kardiovaskuläres Implantat (siehe Figuren 4 und 5), in einen Objektaufnahmebehälter 14 einer Behältereinrichtung 12 eingebracht und fixiert. Beispielsweise wird ein Stent nach Einbringen in den Objektaufnahmebehälter 14 durch einen Ballonkatheter aufgefaltet und an die Wände des Objektaufnahmebehälters 14 gepresst. Gemäß einem weiteren Ausführungsbeispiel wird das eingebrachte Testobjekt 30 durch eine Haltenadel in dem Objektaufnahmebehälter 14 fixiert. Bei einem im Wesentlichen U-förmigen Schlauch wird das kardiovaskuläre Implantat bevorzugt mittig zwischen den ersten und zweiten Enden des Objektaufnahmebehälters 14 fixiert.

Der Objektaufnahmebehälter 14 oder die Behältereinrichtung 12 wird mit Blut gefüllt, so dass eine Blutsäule 20 das Testobjekt 30 vollständig durchströmen und/oder umströmen kann. Erfindungsgemäß hat das Testobjekt 30 während der Expositionszeit keinen Kontakt zur Umgebungsluft, zu einem Druckgas 46 oder einem anderweitig als Puffer zur Druckerzeugungseinrichtung verwendeten inerten Fluid.

An dem ersten Ende 16 des Objektaufnahmebehälters 14 wird zu einem ersten Zeitpunkt ein Überdruck (Pfeil 42 in Figur 4) durch die Druckerzeugungseinrichtung 40 erzeugt. Durch den Überdruck wird die Blutsäule 20 vom ersten Ende 16 zum zweiten Ende 17 des Objektaufnahmebehälters 14 hin getrieben. Zu einem zweiten Zeitpunkt übt ein zweiter Druckerzeuger der Druckerzeugungseinrichtung am zweiten Ende 17 einen Überdruck 42 auf die Blutsäule 20 aus, so dass die Blutsäule 20 vom zweiten Ende 17 zum ersten Ende 16 hin (zurück)getrieben wird (Pfeil 42 in Figur 5). Das abwechselnde Anlegen eines Überdrucks am ersten Ende 16 und am zweiten Ende 17 des Objektaufnahmebehälters 14 wird mit einer festen Frequenz, beispielsweise zwischen 1 Hz und 2 Hz, periodisch wiederholt. Der Objektaufnahmebehälter 14, beispielsweise aus PVC, ist mit so viel Blut befüllt, dass die im Schlauch entstehende Blutsäule 20 einem Vielfachen der Länge des Testobjekts 30 entspricht. Das Testobjekt 30 ist zu jedem Zeitpunkt einer Strömungsperiode vollständig mit Blut benetzt.

Bei Betrieb der Druckerzeugungseinrichtung 40 erfolgt keine mechanische Verformung von in Blutkontakt befindlichen Komponenten der Vorrichtung, wodurch das im Objektaufnahmebehälter 14, beispielsweise einem Schlauch, befindliche Blut schonend und weitestgehend scherratenfrei über das fixierte Testobjekt 30 getrieben wird. Die Strömungsgeschwindigkeit des Blutes kann über die Höhe des Druckunterschiedes, insbesondere des wechselweise am ersten Ende 16 und am zweiten Ende 17 des Objektaufnahmebehälters 14 angelegten Überdrucks (Pfeil 42 in Figuren 4 und 5), auf beiden Seiten reguliert werden.

Der Zyklus bzw. die periodische Druckerzeugung wird wiederholt, bis die gewünschte Expositionszeit von mindestens einer Stunde und höchstens sechs Stunden, beispielsweise zwischen zwei und drei Stunden, erreicht ist. Anschließend werden relevante Blutparameter wie beispielsweise Hämolyse, die Degradierung des von-Willebrand-Faktors und/oder die Aktivierung von Thrombozyten mittels einer Untersuchungseinrichtung 50 bestimmt. Die schematisch gezeigte Untersuchungseinrichtung 50 kann für verschiedene Untersuchungsmethoden oder Assays, wie z. B. immunbiochemische Assays, spektroskopische Analysen und/oder mikroskopische Analysen, ausgelegt sein.

In einer weiteren vorteilhaften Ausgestaltung wird die Belegungsdichte des Testobjekts 30 mit Blutbestandteilen anhand von Färbetechniken und/oder spektroskopischen Methoden bestimmt. Alternativ oder ergänzend kann in einer weiteren vorteilhaften Ausgestaltung während der Exposition die Anlagerung von Blutbestandteilen an das Testobjekt 30 mittels einer weiteren Untersuchungseinrichtung 51 (siehe Figur 1), beispielsweise einer Kamera und/oder eines Spektroskops, beobachtet werden.

Figur 2 zeigt ein weiteres Ausführungsbeispiel einer Vorrichtung 10 zur in-vitro Untersuchung der Wechselwirkung von Blut mit einem Testobjekt, in welcher die Behältereinrichtung 12 mehrere parallel verbundene Objektaufnahmebehälter 14 umfasst. Gemäß dem in Figur 2 dargestellten Ausführungsbeispiel umfasst die Vorrichtung eine Druckerzeugungseinrichtung 40 mit einem ersten Druckerzeuger, der parallel auf die ersten Enden 16 der mehreren Objektaufnahmebehälter 14 wirkt. Alternativ oder ergänzend kann in gleicher Weise ein (nicht dargestellter) zweiter Druckerzeuger der Druckerzeugungseinrichtung auf die zweiten Enden 17 der Objektaufnahmebehälter wirken.

Figur 3 zeigt ein weiteres Ausführungsbeispiel einer Vorrichtung 10 zur in-vitro Untersuchung der Wechselwirkung von Blut mit am Testobjekt, in welcher die Behältereinrichtung 12 mehrere nebeneinander angeordnete Objektaufnahmebehälter 14 umfasst und jeder Objektaufnahmebehälter 14 an seinem ersten Ende 16 mit einem einzeln steuerbaren Druckerzeuger der Druckerzeugungseinrichtung 40 verbunden ist. Alternativ oder ergänzend können weitere (nicht gezeigte) Druckerzeuger der Druckerzeugungseinrichtung mit den zweiten Enden 17 der Objektaufnahmebehälter 14 verbunden sein.

Die Ausführungsbeispiele der Figuren 2 und 3 sind kombinierbar, indem beispielsweise die ersten Enden 16 mit einem einzigen, auf alle Objektaufnahmebehälter 14 wirkenden Druckerzeuger (z. B. Druckkolben) und jedes der zweiten Enden 17 mit einem einzelnen, jeweils auf den zugehörigen Objektaufnahmebehälter 14 wirkenden Druckerzeuger (z. B. Druckkolben) verbunden ist.

Figur 4 und Figur 5 zeigen schematisch einen Objektaufnahmebehälter 14 einer Behältereinrichtung 12 mit einem Testobjekt 30. Der Objektaufnahmebehälter 14 erstreckt sich in der Praxis, wie in den Figuren 1 bis 3 gezeigt, vorzugsweise U-förmig mit nach oben gerichteten Enden 16, 17. Es kann in der Praxis aber auch die illustrierte gerade Form des Objektaufnahmebehälters 14 vorgesehen sein. Der Objektaufnahmebehälter 14 weist eine Länge 18 von z. B. 70 cm und einen Innendurchmesser 19 von z. B. 3 mm auf. Der Objektaufnahmebehälter 14 ist in den Figuren 4 und 5 mit einer Blutsäule 20 gefüllt und ein Testobjekt 30 im Wesentlichen mittig im Objektaufnahmebehälter 14 fixiert. An dem ersten und zweiten Ende 16 bzw. 17 des Objektaufnahmebehälters 14 befindet sich gemäß diesem Ausführungsbeispiel ein Druckgas 46. Alternativ oder ergänzend kann die Blutsäule 20 durch ein anderweitiges inertes Fluid 46 bezüglich eines Druckerzeugers einer Druckerzeugungseinrichtung 40 gepuffert sein.

In Figur 4 befindet sich die Blutsäule 20 zu Beginn eines ersten Betriebszustands der Vorrichtung 10 nahe am ersten Ende 16 des Objektaufnahmebehälters 14. Ein Druckerzeuger (nicht gezeigt) der Druckerzeugungseinrichtung am ersten Ende 16 erzeugt einen Überdruck im Druckgas 46 (siehe Pfeil 42), um die Blutsäule 20 zum zweiten Ende 17 zu treiben. Am Ende des ersten Betriebszustands befindet sich die Blutsäule 20, wie in Figur 5 gezeigt, nahe dem zweiten Ende 17 des Objektaufnahmebehälters 14. In einem zweiten Betriebszustand der Vorrichtung 10 erzeugt nun ein zweiter Druckerzeuger (nicht gezeigt) der Druckerzeugungseinrichtung am zweiten Ende 17 einen Überdruck im Druckgas 46 (siehe Pfeil 42), der die Blutsäule 20 zum ersten Ende 16 zurücktreibt. Sobald sich die Blutsäule 20 wieder nahe am ersten Ende 16, wie in Figur 4 gezeigt, befindet, wird wieder zum ersten Betriebszustand der Vorrichtung 10 gewechselt.

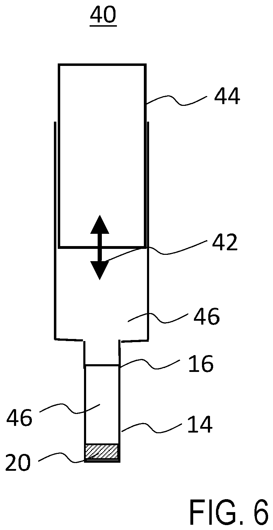

Figur 6 zeigt eine beispielhafte Ausführungsform einer Druckerzeugungseinrichtung 40, die an einem ersten Ende 16 eines Objektaufnahmebehälters 14 angeordnet ist. Die Druckerzeugungseinrichtung 40 umfasst einen Druckkolben 44, der einen Überdruck (siehe Pfeil 42) auf ein sich in einem geschlossenen Hohlraum befindendes Druckgas 46 oder anderweitiges inertes Fluid erzeugt. Alternativ oder ergänzend kann eine weitere, vorzugsweise baugleiche, Druckerzeugungseinrichtung an einem zweiten Ende 17 des Objektaufnahmebehälters 14 angeordnet sein.

Der erfindungsmäßige Pumpmechanismus für dynamische Tests zur Wechselwirkung von Blut mit dem Testobjekt 30 erzeugt vorteilhafterweise einen scherratenarmen Vortrieb des Blutes über das Testobjekt 30. Weiterhin vorteilhaft ermöglicht das erfindungsgemäße Verfahren Untersuchungen mit sehr kleinen Probenvolumina, beispielsweise geringen Blutmengen zwischen 0,5 ml und 10 ml und kleinen Durchmessern des Testobjekts 30. Der Einfluss der Geometrie des Testobjekts 30, beispielsweise eines Implantats, auf strömungsabhängige Blutreaktionen kann präzise und robust abgebildet werden.

Gleichzeitig können physiologische Strömungsprofile (d. h. hohe Fließraten und/oder eine gepulste Strömung) kontrolliert appliziert werden, was die Grundlage für die Erhebung robuster, reproduzierbarer und somit brauchbarer Daten bildet. Zudem ist der Mechanismus einfach und kostengünstig, leicht parallelisierbar, sowie der jeweiligen Probengeometrie, beispielsweise der Geometrie des Testobjekts 30, flexibel anpassbar. Damit unterscheidet er sich deutlich vom jahrzehntelang mit den vorstehend beschriebenen Beschränkungen eingesetzten Stand der Technik, z.B. dem "Chandler-Loop", der Rollerpumpe (Quetschpumpe) und/oder dem "Hemobile".

Obwohl die Erfindung in Bezug auf exemplarische Ausführungsbeispiele beschrieben worden ist, ist es für einen Fachmann ersichtlich, dass verschiedene Änderungen vorgenommen werden können und Äquivalente als Ersatz verwendet werden können. Ferner können viele Modifikationen vorgenommen werden, um eine bestimmte Behältereinrichtung oder eine bestimmte Druckerzeugungseinrichtung an die Lehre der Erfindung anzupassen. Folglich ist die Erfindung nicht auf die offenbarten Ausführungsbeispiele beschränkt, sondern umfasst alle Ausführungsbeispiele, die in den Bereich der beigefügten Ansprüche fallen.

Die in der vorstehenden Beschreibung, den Zeichnungen und den Ansprüchen offenbarten Merkmale der Erfindung können sowohl einzeln als auch in Kombination oder Unterkombination für die Verwirklichung der Erfindung in ihren verschiedenen Ausgestaltungen von Bedeutung sein.

## Patentansprüche

1. Verfahren zur in-vitro Untersuchung der Wechselwirkung von Blut mit einem Testobjekt (30), umfassend die Schritte
- Bereitstellung des Testobjekts (30) und des Blutes in einer Behältereinrichtung (12), wobei das Testobjekt (30) in der Behältereinrichtung (12) fixiert ist und das Blut als eine Blutsäule (20) entlang einer Längsrichtung (18) der Behältereinrichtung (12) angeordnet ist,
- Erzeugung einer Blutströmung in der Behältereinrichtung (12) für eine vorbestimmte Expositionszeit mittels einer Druckerzeugungseinrichtung (40), wobei die Blutströmung am Testobjekt (30) vorbeitritt und/oder durch eine Öffnung des Testobjekts (30) durchtritt, und
- Untersuchung des Blutes zur Ermittlung mindestens eines Blutparameters, der von einer Wechselwirkung des Blutes mit dem Testobjekt (30) abhängig ist, wobei
- mit der Druckerzeugungseinrichtung (40) an mindestens einem Ende (16, 17) der Behältereinrichtung (12) ein äußerer, veränderlicher Druck auf die Blutsäule (20) ausgeübt wird, so dass die Blutströmung mit einer alternierenden Strömungsrichtung entlang der Längsrichtung (18) der Behältereinrichtung (12) erzeugt wird,
**dadurch gekennzeichnet, dass**
- der Druck durch die Druckerzeugungseinrichtung (40) an mindestens einem der Enden (16, 17) der Behältereinrichtung (12) durch eine Applikation eines direkt auf das Blut wirkenden, mit dem Blut nicht wechselwirkenden, Druckgases (46) und/oder eines direkt auf das Blut wirkenden, mit dem Blut nicht wechselwirkenden, flüssigen Fluids (46), dessen Dichte geringer ist als die Dichte des Blutes, ausgeübt wird.

2. Verfahren gemäß Anspruch 1, wobei der Druck wechselweise an beiden Enden (16, 17) der Behältereinrichtung (12) als Überdruck ausgeübt wird.

3. Verfahren gemäß einem der vorhergehenden Ansprüche, bei der
- der Druck durch die Druckerzeugungseinrichtung (40) durch die Applikation des auf das Blut wirkenden Druckgases (46) ausgeübt wird, das Luft oder ein Edelgas umfasst.

4. Verfahren gemäß einem der vorhergehenden Ansprüche, bei dem die Behältereinrichtung (12) umfasst
- einen einzigen Objektaufnahmebehälter (14), oder
- mehrere Objektaufnahmebehälter (14), wobei die Druckerzeugungseinrichtung (40) auf alle Objektaufnahmebehälter (14) gemeinsam oder auf jeden Objektaufnahmebehälter (14) einzeln wirkt.

5. Verfahren gemäß einem der vorhergehenden Ansprüche, mit mindestens einem der Merkmale
- das Testobjekt (30) ist ein kardiovaskuläres Implantat oder ein Medizinprodukt, und
- die Expositionszeit beträgt mindestens eine Stunde und/oder höchstens sechs Stunden.

6. Verfahren gemäß einem der vorhergehenden Ansprüche, bei dem
- der mindestens eine Blutparameter mindestens eines aus Hämolyse, Degradierung des von-Willebrand-Faktors und dem Aktivierungszustand von Thrombozyten ist.

7. Verfahren gemäß einem der vorhergehenden Ansprüche, umfassend den Schritt
- Untersuchung des Testobjekts (30) zur Ermittlung einer Belegungsdichte mit Blutbestandteilen, insbesondere einer Anlagerung von Thrombozyten und/oder Proteinen.

8. Vorrichtung (10), die zur in-vitro Untersuchung der Wechselwirkung von Blut mit einem Testobjekt (30) eingerichtet ist, umfassend
- eine Behältereinrichtung (12), die dazu ausgebildet ist, das zu untersuchende Testobjekt (30) in einer fixierten Anordnung und ein Testvolumen von Blut in Form einer Blutsäule (20) entlang einer Längsrichtung (18) einer Behältereinrichtung (12) aufzunehmen, und
- eine Druckerzeugungseinrichtung (40), die dazu ausgebildet ist, in der Behältereinrichtung (12) eine Blutströmung zu erzeugen, die am Testobjekt (30) vorbeitritt und/oder durch eine Öffnung des Testobjekts (30) durchtritt,
- die Behältereinrichtung (12) zwei Enden (16, 17) aufweist, wobei die Druckerzeugungseinrichtung (40) mit mindestens einem der Enden (16, 17) gekoppelt ist, und
- die Druckerzeugungseinrichtung (40) dazu ausgebildet ist, an mindestens einem der Enden der Behältereinrichtung (12) einen äußeren, veränderlichen Druck auf die Blutsäule (20) auszuüben, so dass die Blutströmung mit einer alternierenden Strömungsrichtung entlang der Längsrichtung (18) der Behältereinrichtung (12) erzeugt werden kann,
**dadurch gekennzeichnet, dass**
- die Druckerzeugungseinrichtung (40) dazu ausgebildet ist, den Druck an mindestens einem der Enden (16, 17) der Behältereinrichtung (12) durch eine Applikation eines direkt auf das Blut wirkenden Druckgases (46) und/oder eines direkt auf das Blut wirkenden, mit dem Blut nicht wechselwirkenden, flüssigen Fluids (46), dessen Dichte geringer ist als die Dichte des Blutes, auszuüben.

9. Vorrichtung (10) gemäß Anspruch 8, bei der
- die Druckerzeugungseinrichtung (40) dazu ausgebildet ist, den Druck als Überdruck wechselweise an beiden Enden (16, 17) der Behältereinrichtung (12) auszuüben.

10. Vorrichtung (10) gemäß Anspruch 8 oder 9, bei der
- die Druckerzeugungseinrichtung (40) dazu ausgebildet ist, den Druck durch die Applikation des auf das Blut wirkenden Druckgases (46) auszuüben, Luft oder ein Edelgas umfasst.

11. Vorrichtung (10) gemäß einem der Ansprüche 8 bis 10, bei der
- die Behältereinrichtung (12) in der Längsausdehnung (18) eine Länge aufweist, die mindestens so lang ist, dass das Testobjekt (30) zu jedem Zeitpunkt der Blutströmung mit alternierender Strömungsrichtung von Blut benetzt ist.

12. Vorrichtung (10) gemäß einem der Ansprüche 8 bis 11, bei der die Behältereinrichtung (12) umfasst
- einen einzigen Objektaufnahmebehälter (14), oder
- mehrere Objektaufnahmebehälter (14), wobei die Druckerzeugungseinrichtung (40) auf alle Objektaufnahmebehälter (14) gemeinsam oder auf jeden Objektaufnahmebehälter (14) einzeln wirkt.

13. Vorrichtung (10) gemäß einem der Ansprüche 8 bis 12, bei der
- die Behältereinrichtung (12) oder der mindestens eine Objektaufnahmebehälter (14) quer zu der Längsausdehnung (18) eine Innenquerschnittsdimension (19) aufweist, die an eine Außenquerschnittsdimension des Testobjekts (30) angepasst ist.

14. Vorrichtung (10) gemäß einem der Ansprüche 8 bis 13, bei der
- das Testobjekt (30) in der Behältereinrichtung (12) oder dem mindestens einen Objektaufnahmebehälter (14) mittels einer Festhaltevorrichtung fixiert ist.

15. Vorrichtung (10) gemäß einem der Ansprüche 8 bis 14, mit mindestens einem der Merkmale
- die Behältereinrichtung (12) ist zur Aufnahme des Testobjekts (30) in Gestalt eines kardiovaskulären Implantats oder eines Medizinprodukts eingerichtet, und
- die Behältereinrichtung (12) oder der mindestens eine Objektaufnahmebehälter (14) umfasst einen geradlinigen oder U-förmig gebogenen Schlauch oder ein geradliniges oder U-förmig gebogenes Rohr, insbesondere aus einem Kunststoff.

## Claims

1. Method for in vitro investigating the interaction of blood with a test object (30), comprising the steps of
- providing the test object (30) and the blood in a container device (12), wherein the test object (30) is fixed in the container device (12) and the blood is arranged as a blood column (20) along a longitudinal direction (18) of the container device (12),
- generating a blood flow in the container device (12) for a predetermined exposure time by means of a pressure generating device (40), the blood flow passing the test object (30) and/or passing through an opening of the test object (30), and
- investigating the blood to determine at least one blood parameter which is dependent on an interaction of the blood with the test object (30), wherein
- an external, variable pressure is exerted on the blood column (20) with the pressure generating device (40) at at least one end (16, 17) of the container device (12), so that the blood flow is generated with an alternating flow direction along the longitudinal direction (18) of the container device (12), **characterized in that**
- the pressure is exerted by the pressure generating device (40) at at least one of the ends (16, 17) of the container device (12) by an application of a pressurized gas (46) acting directly on the blood and not interacting with the blood and/or a liquid fluid (46) acting directly on the blood and not interacting with the blood, the density of which is lower than the density of the blood.

2. Method according to claim 1, wherein the pressure is alternately applied to both ends (16, 17) of the container device (12) as an overpressure.

3. Method according to one of the preceding claims, in which
- the pressure is exerted by the pressure generating device (40) by the application of the pressurized gas (46) acting on the blood, the pressurized gas (46) comprising air or a noble gas.

4. Method according to one of the preceding claims, in which the container device (12) comprises
- a single object receiving container (14), or
- multiple object receiving containers (14), wherein the pressure generating device (40) acts on all object receiving containers (14) together or on each object receiving container (14) individually.

5. Method according to one of the preceding claims, comprising at least one of the features
- the test object (30) is a cardiovascular implant or a medical device, and
- the exposure time is at least one hour and/or at most six hours.

6. Method according to one of the preceding claims, wherein
- the at least one blood parameter is at least one of hemolysis, degradation of von Willebrand factor and activation state of thrombocytes.

7. Method according to one of the preceding claims, comprising the step of
- Investigating the test object (30) to determine an occupancy density with blood components, in particular an accumulation of thrombocytes and/or proteins.

8. Device (10) being adapted for in vitro investigating of the interaction of blood with a test object (30), comprising
- a container device (12) configured to receive the test object (30) to be examined in a fixed arrangement and a test volume of blood in the form of a blood column (20) along a longitudinal direction (18) of a container device (12), and
- a pressure generating device (40) configured to generate in the container device (12) a flow of blood passing the test object (30) and/or passing through an opening of the test object (30), wherein
- the container device (12) has two ends (16, 17), wherein the pressure generating device (40) is coupled to at least one of the ends (16, 17), and
- the pressure generating device (40) is configured to exert an external, variable pressure on the blood column (20) at at least one of the ends of the container device (12), such that the blood flow can be generated with an alternating flow direction along the longitudinal direction (18) of the container device (12)
**characterized in that**
- the pressure generating device (40) is configured to exert the pressure at at least one of the ends (16, 17) of the container device (12) by an application of a pressurized gas (46) acting directly on the blood and/or a liquid fluid (46) acting directly on the blood and not interacting with the blood, the density of which fluid is lower than the density of the blood.

9. Device (10) according to claim 8, in which
- the pressure generating device (40) is configured to exert the pressure as an overpressure alternately at both ends (16, 17) of the container device (12).

10. Device (10) according to claim 8 or 9, in which
- the pressure generating device (40) is configured to exert the pressure by the application of the pressurized gas (46) acting on the blood, the pressurized gas (46) comprising air or a noble gas.

11. Device (10) according to one of claims 8 to 10, in which
- the container device (12) has a length in the longitudinal extension (18) at least long enough to wet the test object (30) with blood at any time of the blood flow with alternating flow direction.

12. Device (10) according to one of claims 8 to 11, wherein the container device (12) comprises
- a single object receiving container (14), or
- a plurality of object receiving containers (14), wherein the pressure generating device (40) acts on all object receiving containers (14) together or on each object receiving container (14) individually.

13. Device (10) according to one of claims 8 to 12, wherein
- the container device (12) or the at least one object receiving container (14) has, transversely to the longitudinal extension (18), an internal cross-sectional dimension (19) adapted to an external cross-sectional dimension of the test object (30).

14. Device (10) according to one of claims 8 to 13, wherein
- the test object (30) is fixed in the container device (12) or the at least one object receiving container (14) by means of a retaining device.

15. Device (10) according to one of claims 8 to 14, having at least one of the features
- the container device (12) is adapted to receive the test object (30) in the form of a cardiovascular implant or a medical device, and
- the container device (12) or the at least one object receiving container (14) comprises a straight or U-shaped bent hose or a straight or U-shaped bent tube, in particular made of a plastic.

## Revendications

1. Procédé d'examen *in vitro* de l'interaction du sang avec un objet de test (30), comprenant les étapes
- de fourniture de l'objet de test (30) et du sang dans un système de contenant (12), dans lequel l'objet de test (30) est fixé dans le système de contenant (12) et le sang est disposé comme une colonne de sang (20) le long d'un sens longitudinal (18) du système de contenant (12),
- de production d'un écoulement de sang dans le système de contenant (12) pour un temps d'exposition prédéfini au moyen d'un système de production de pression (40), dans lequel l'écoulement de sang longe l'objet de test (30) et/ou traverse une ouverture de l'objet de test (30), et
- d'examen du sang pour déterminer au moins un paramètre du sang, qui dépend d'une interaction du sang avec l'objet de test (30), dans lequel
- une pression extérieure variable est exercée sur la colonne de sang (20) avec le système de production de pression (40) sur au moins une extrémité (16, 17) du système de contenant (12) de sorte que l'écoulement de sang est produit avec un sens d'écoulement qui alterne le long du sens longitudinal (18) du système de contenant (12),
**caractérisé en ce que**
- la pression est exercée par le système de production de pression (40) sur au moins une des extrémités (16, 17) du système de contenant (12) par une application d'un gaz de pression (46) agissant directement sur le sang, n'interagissant pas avec le sang, et/ou d'un fluide (46) liquide agissant directement sur le sang, n'interagissant pas avec le sang, dont la densité est inférieure à la densité du sang.

2. Procédé selon la revendication 1, dans lequel la pression est exercée en tant que surpression en alternance sur les deux extrémités (16, 17) du système de contenant (12).

3. Procédé selon l'une quelconque des revendications précédentes, où
- la pression est exercée par le système de production de pression (40) par l'application du gaz de pression (46) agissant sur le sang, qui comprend de l'air ou un gaz noble.

4. Procédé selon l'une quelconque des revendications précédentes, où le système de contenant (12) comprend
- un unique contenant de réception d'objet (14), ou
- plusieurs contenants de réception d'objet (14), dans lequel le système de production de pression (40) agit sur tous les contenants de réception d'objet (14) conjointement ou sur chaque contenant de réception d'objet (14) individuellement.

5. Procédé selon l'une quelconque des revendications précédentes, avec au moins une des caractéristiques
- l'objet de test (30) est un implant cardiovasculaire ou un produit médical, et
- le temps d'exposition est d'au moins une heure et/ou de six heures au maximum.

6. Procédé selon l'une quelconque des revendications précédentes, où
- l'au moins un paramètre du sang est au moins un parmi une hémolyse, la dégradation du facteur de von Willebrand et l'état d'activation de thrombocytes.

7. Procédé selon l'une quelconque des revendications précédentes, comprenant l'étape
- de l'examen de l'objet de test (30) pour déterminer une densité d'occupation avec des constituants du sang, en particulier d'une accumulation de thrombocytes et/ou de protéines.

8. Dispositif (10), qui est mis au point pour l'examen *in vitro* de l'interaction du sang avec un objet de test (30), comprenant
- un système de contenant (12), qui est réalisé pour recevoir l'objet de test (30) à examiner dans un agencement fixé et un volume de test du sang sous la forme d'une colonne de sang (20) le long d'un sens longitudinal (18) d'un système de contenant (12), et
- un système de production de pression (40), qui est réalisé pour produire un écoulement de sang dans le système de contenant (12), qui longe l'objet de test (30) et/ou traverse une ouverture de l'objet de test (30),
- le système de contenant (12) présente deux extrémités (16, 17), dans lequel le système de production de pression (40) est couplé à au moins une des extrémités (16, 17), et
- le système de production de pression (40) est réalisé pour exercer une pression extérieure variable sur la colonne de sang (20) sur au moins une des extrémités du système de contenant (12) de sorte que l'écoulement de sang peut être produit avec un sens d'écoulement qui alterne le long du sens longitudinal (18) du système de contenant (12),
**caractérisé en ce que**
- le système de production de pression (40) est réalisé pour exercer la pression sur au moins une des extrémités (16, 17) du système de contenant (12) par une application d'un gaz de pression (46) agissant directement sur le sang et/ou d'un fluide (46) liquide agissant directement sur le sang, n'interagissant pas avec le sang, dont la densité est inférieure à la densité du sang.

9. Dispositif (10) selon la revendication 8, où
- le système de production du sang (40) est réalisé pour exercer la pression en tant que surpression en alternance sur les deux extrémités (16, 17) du système de contenant (12).

10. Dispositif (10) selon la revendication 8 ou 9, où
- le système de production de pression (40) est réalisé pour exercer la pression par l'application du gaz de pression (46) agissant sur le sang, qui comprend de l'air ou un gaz noble.

11. Dispositif (10) selon l'une quelconque des revendications 8 à 10, où
- le système de contenant (12) présente dans l'extension longitudinale (18) une longueur, qui est au moins si grande que l'objet de test (30) est humidifié par du sang à chaque moment de l'écoulement de sang avec un sens d'écoulement qui alterne.

12. Dispositif (10) selon l'une quelconque des revendications 8 à 11, où le système de contenant (12) comprend
- un unique contenant de réception d'objet (14), ou
- plusieurs contenants de réception d'objet (14), dans lequel le système de production de pression (40) agit sur tous les contenants de réception d'objet (14) conjointement ou sur chaque contenant de réception d'objet (14) individuellement.

13. Dispositif (10) selon l'une quelconque des revendications 8 à 12, où
- le système de contenant (12) ou l'au moins un contenant de réception d'objet (14) présente de manière transversale par rapport à l'extension longitudinale (18) une dimension de section transversale intérieure (19), qui est adaptée à une dimension de section transversale extérieure de l'objet de test (30).

14. Dispositif (10) selon l'une quelconque des revendications 8 à 13, où
- l'objet de test (30) est fixé dans le système de contenant (12) ou l'au moins un contenant de réception d'objet (14) au moyen d'un dispositif d'immobilisation.

15. Dispositif (10) selon l'une quelconque des revendications 8 à 14, avec au moins une des caractéristiques
- le système de contenant (12) est mis au point pour recevoir l'objet de test (30) sous la forme d'un implant cardiovasculaire ou d'un produit médical, et
- le système de contenant (12) ou l'au moins un contenant de réception d'objet (14) comprend un tuyau flexible rectiligne ou cintré en forme de U ou un tube rectiligne ou cintré en forme de U, en particulier composé d'une matière plastique.
